# FASCICULE DE BREVET EUROPEEN

(11) **EP 3 526 445 B1**
(45) Date de publication et mention de la délivrance du brevet: **09.12.2020**
(21) Numéro de dépôt: 17781527.1
(22) Date de dépôt: 17.10.2017
(51) Int. Cl.: E21B 49/00, E21B 21/06

(54) **CANNE DE PRÉLÈVEMENT DE FLUIDE**
FLUIDPROBENAHMESONDE
FLUID SAMPLING PROBE

(30) Priorité: 17.10.2016 FR 1660025
(43) Date de publication de la demande: 21.08.2019
(73) Titulaire: Excellence Logging France, 95110 Sannois (FR)
(72) Inventeur: SOURICE, Louis, 17137 Niel Sur Mer (FR); GIRAUDET, Samuel, 92400 Courbevoie (FR)
(74) Mandataire: Lavoix
(86) Numéro de dépôt international: PCT/EP2017/076475
(87) Numéro de publication internationale: WO 2018/073249

(56) Documents cités:
- US-A1- 2011 308 787
- US-A1- 2012 160 514
- US-A1- 2013 032 527
- US-A1- 2013 062 051
- US-A1- 2014 131 031
- US-A1- 2014 144 625
- US-A1- 2014 216 176

## Description

La présente invention concerne une canne de prélèvement d'un fluide de forage comprenant :
- un manche s'étendant selon un axe ;
- une tête de prélèvement située à une première extrémité du manche, la tête définissant un espace interne débouchant vers l'extérieur par une ouverture, la tête comprenant un dispositif de filtration comprenant une paroi filtrante disposée à travers l'ouverture ; et
- une conduite d'extraction s'étendant dans le manche et débouchant dans l'espace interne de la tête. L'invention concerne également un système de prélèvement de fluide et une installation de forage pétrolier.

Au cours d'une opération de forage pétrolier, des fluides sont injectés en permanence depuis les installations de surface vers le fond du puits, notamment pour refroidir et lubrifier les outils de forage au fond du puits. Ces fluides remontent ensuite vers la surface, entrainant avec eux les débris de roches arrachés lors du forage. De plus, les fluides sont exposés aux formations rocheuses traversées, et peuvent se charger en hydrocarbures selon les contenus des formations rencontrés.

Il est connu d'analyser les fluides de forage en sortie de puits afin d'obtenir des informations sur la nature des formations rocheuses et de leur contenu, notamment en hydrocarbures et en gaz dissous.

Les dispositifs connus d'analyse de fluides de forage comprennent généralement un dispositif de prélèvement inséré dans le flux des fluides de forage en sorite de puits, et relié à une pompe par un tuyau flexible. La pompe et les dispositifs d'analyse de fluide sont généralement situés loin de la conduite de circulation, pour des raisons de sécurité, mais aussi afin perturber le moins possible les opérations de forage et de pompage.

Compte tenu de la nature des fluides, il est nécessaire pour ce dispositif de présenter un filtre d'entrée, afin d'éviter que des débris de roches ne viennent obturer la conduite de prélèvement. De plus, comme les fluides de forage contiennent beaucoup de particules solides, le filtre doit être nettoyé régulièrement afin d'éviter que celui-ci ne se bouche.

Il est connu, par exemple dans le document US 5,090,256, d'utiliser une lame de nettoyage tournante pour nettoyer en permanence le filtre pendant l'extraction de fluides de forage. Cette lame est mise en mouvement par un moteur situé loin de la conduite de circulation du fluide, le couple moteur étant transmis par un câble Bowden.

Ce dispositif comprend donc deux tubages distincts et présente une grande rigidité, ce qui complique son insertion dans une conduite de circulation fermée. De plus, le câble Bowden est sujet à une usure importante, ce qui présente des risques de dégâts mécaniques.

Le document US 2014/0216176 décrit un dispositif de prélèvement amélioré sous la forme d'une canne métallique fermée, facilement insérable dans une conduite de circulation fermée de manière étanche. Ce dispositif comprend également un filtre nettoyé par une lame tournante entrainée par un câble Bowden.

Le document US2013/062051 décrit un outil au fond de puits, comprenant une paroi de filtration et un piston apte à parcourir en translation une surface interne de la paroi.

Le document US2014/144625 décrit un outil au fond de puits, comprenant une paroi filtrante et une lame élastique apte à parcourir une surface externe de la paroi.

Ce dispositif peut encore être amélioré. La présente invention a pour but de fournir une canne de prélèvement présentant des contraintes d'utilisation réduites par rapport aux dispositifs antérieurs.

Ainsi, l'invention a pour objet une canne de prélèvement du type précité, caractérisée en ce que le dispositif de filtration comprend une lame de nettoyage apte à parcourir une face externe de la paroi filtrante, la lame étant montée mobile en translation selon une direction parallèle à l'axe.

Selon des modes de réalisation particuliers, la canne selon l'invention présente l'une ou plusieurs des caractéristiques suivantes, prise(s) isolément ou selon toute combinaison techniquement possible :
- le dispositif de filtration comprend un vérin apte à déplacer la lame sur la face externe de la paroi filtrante ;
- le dispositif de filtration comprend au moins une conduite pneumatique contenue dans le manche et débouchant dans le vérin ;
- la lame présente une ouverture centrale ;
- la lame présente des surfaces de contact avec la paroi filtrante uniquement au niveau des bords latéraux de la lame ;
- la paroi filtrante présente une pluralité d'orifices présentant une première section transversale située au voisinage de l'espace interne, d'aire supérieure à l'aire d'une deuxième section transversale située à l'écart extérieurement de la première section transversale ;
- les orifices présentent une forme tronconique ;
- la lame et la surface filtrante sont assemblées dans des rainures de la tête, le dispositif de filtration comprenant un dispositif de maintien de la lame et de la paroi filtrante, fixé de manière amovible à la tête ;
- la face externe de la paroi filtrante présente une normale sensiblement perpendiculaire à l'axe ; et
- la canne comprend une conduite de réinjection de fluide contenue dans le manche et débouchant dans l'extérieur à travers un orifice de sortie situé au niveau de la tête, à l'opposé de la paroi filtrante.

L'invention a également pour objet un système de prélèvement de fluide, comportant :
- une canne telle que décrite plus haut, la tête de la canne étant destinée à être placée dans un fluide à prélever ;
- une pompe d'extraction reliée fluidiquement à une extrémité libre de la conduite d'extraction de la canne ;
- un dispositif d'analyse du fluide, relié fluidiquement à une sortie de la pompe d'extraction.

Selon un mode de réalisation particulier, le système selon l'invention comprend la caractéristique suivante :
- le système comprend une pompe de réinjection reliée fluidiquement à la conduite de réinjection, l'orifice de sortie étant situé en aval de la paroi filtrante par rapport à un sens de circulation du fluide.

L'invention a également pour objet une installation de forage pétrolier, comprenant un système de prélèvement de fluide tel que décrit plus haut et une conduite dans laquelle circule un fluide de forage, la tête étant insérée dans la conduite et immergée dans le fluide.

Selon des modes de réalisation particuliers, l'installation selon l'invention présente l'une ou plusieurs des caractéristiques suivantes, prise(s) isolément ou selon toute combinaison techniquement possible :
- la tête est orientée dans la conduite de façon à ce que la face externe de la paroi filtrante soit face à un sens de circulation du fluide de forage ; et
- l'installation comprend une pompe d'extraction de fluide, raccordée à la conduite d'extraction, une chambre de séparation des gaz et un dispositif d'analyse des gaz récupérés dans la chambre de séparation.

L'invention a également pour objet une canne de prélèvement d'un fluide de forage comprenant :
- un manche s'étendant selon un axe ;
- une tête de prélèvement située à une première extrémité du manche, la tête définissant un espace interne débouchant vers l'extérieur par une ouverture, la tête comprenant un dispositif de filtration comprenant une paroi filtrante disposée à travers l'ouverture ; et
- une conduite d'extraction s'étendant dans le manche et débouchant dans l'espace interne de la tête,
caractérisée en ce que la canne comprend la canne comprend une conduite de réinjection de fluide contenue dans le manche et débouchant dans l'extérieur à travers un orifice de sortie situé au niveau de la tête, à l'opposé de la paroi filtrante.

La canne ne comprend pas nécessairement une lame de nettoyage.

L'invention sera mieux comprise à la lecture de la description qui va suivre, donnée uniquement à titre d'exemple et faite en référence aux dessins annexés, parmi lesquels :
- la figure 1 est une vue en perspective d'une canne selon l'invention ;
- la figure 2 est une vue en perspective d'une tête de la canne de la figure 1 ;
- la figure 3 est une vue éclatée de la tête de la figure 2 ;
- la figure 4 est une vue en coupe d'un dispositif de filtration de la tête des figures 2 et 3 ;

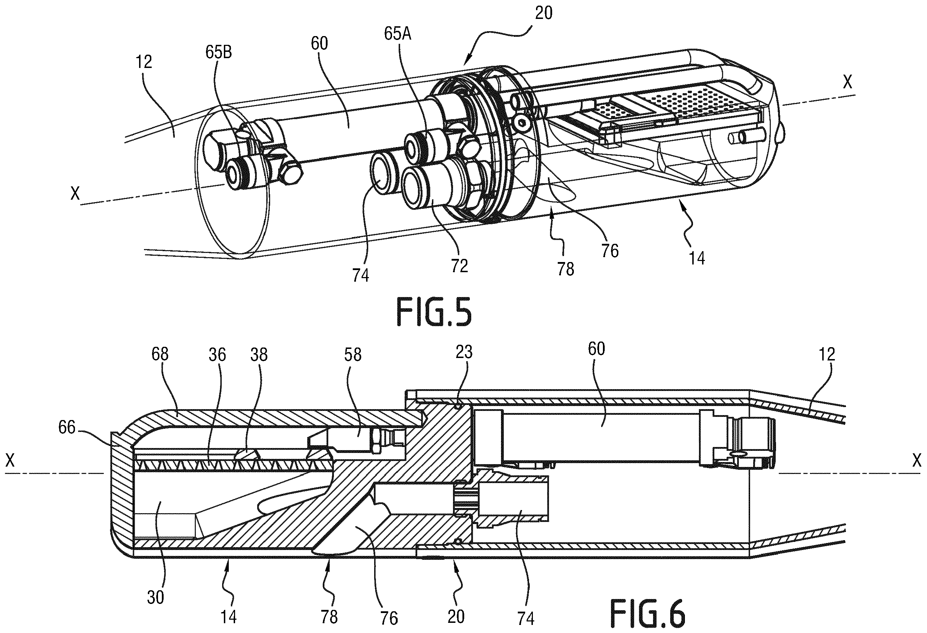

- la figure 5 est une vue arrière de la tête des figures 2 à 4 ;
- la figure 6 est une vue en coupe de la tête des figures 2 à 5 ; et
- la figure 7 est une vue en coupe latérale d'une conduite de circulation recevant la canne de la figure 1.

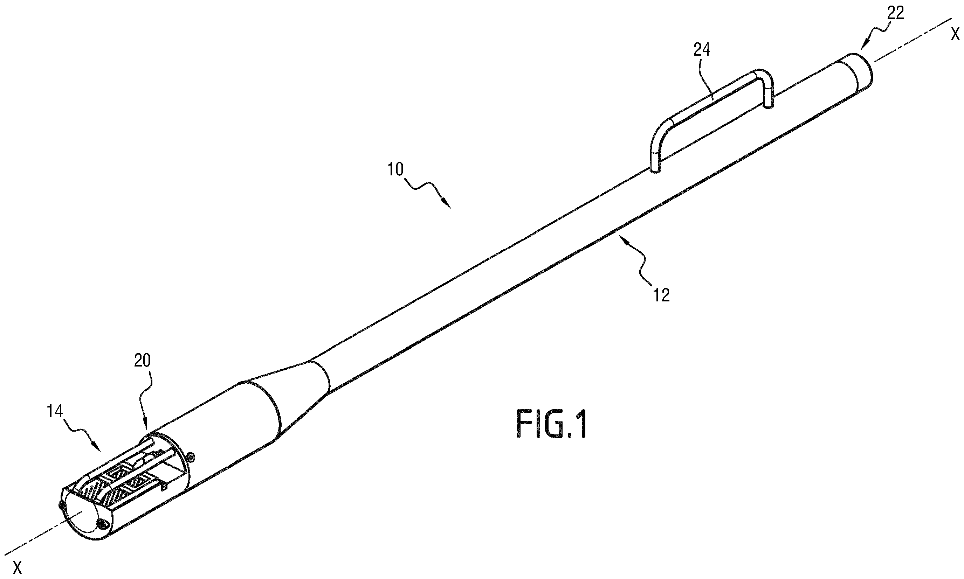

Une canne 10 de prélèvement de fluide selon l'invention est représentée sur la figure 1.

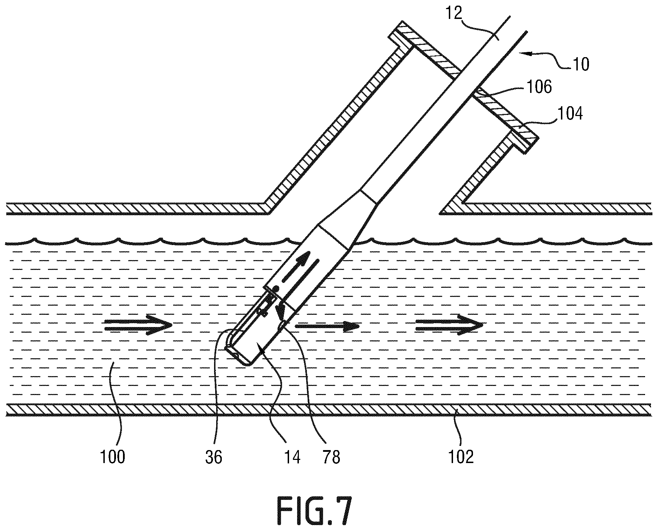

La canne 10 est destinée à être insérée dans une conduite 102, visible sur la figure 7, pour prélever un fluide 100 présente dans la conduite 102. La conduite 102 est par exemple située à la sortie d'un puits en cours de forage, pour récupérer un fluide sortant du puits. En variante, la conduite 102 est située en entrée du puits et reçoit un fluide destiné à être injecté dans le puits.

En référence à la figure 1, la canne 10 comprend un manche 12 et une tête de prélèvement 14 fixée au manche 12, ainsi qu'une conduite d'extraction 16 et une conduite de réinjection 18 contenues dans le manche 12.

Le manche 12 est un tube creux, par exemple métallique. Il présente une première extrémité 20 et une deuxième extrémité 22. Le manche 12 s'étend selon un axe longitudinal X-X.

Le manche 12 définit un volume interne dans lequel s'étendent la conduite d'extraction 16 et la conduite de réinjection 18, de la première extrémité 20 à la deuxième extrémité 22.

Le manche 12 présente un évasement au niveau de la première extrémité 20, dans lequel est emmanchée la tête 14. La tête 14 est munie d'un joint torique 23 situé entre l'évasement de la première extrémité 20 et la tête 14 pour assurer l'étanchéité de la canne 10.

Avantageusement, le manche 12 comporte une poignée 24 proche de la deuxième extrémité 22, afin de faciliter le maniement de la canne 10 par un utilisateur.

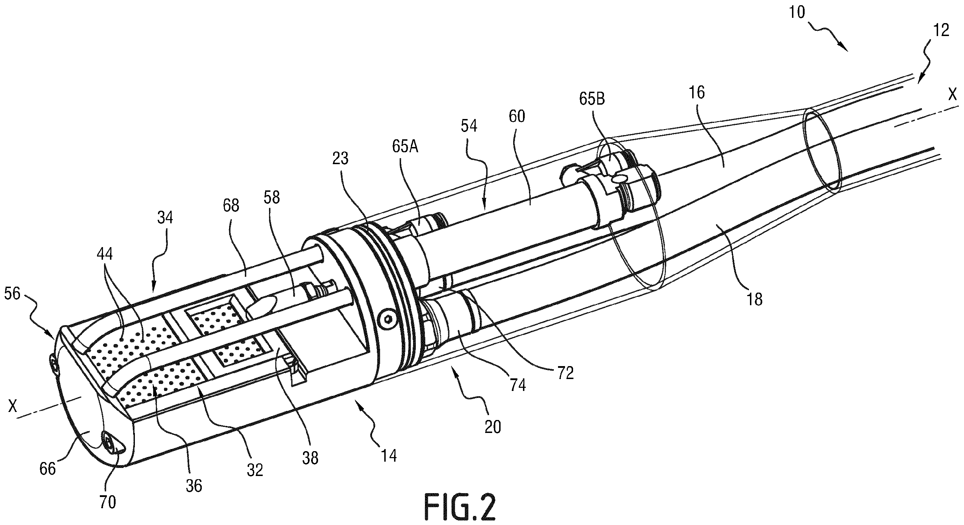

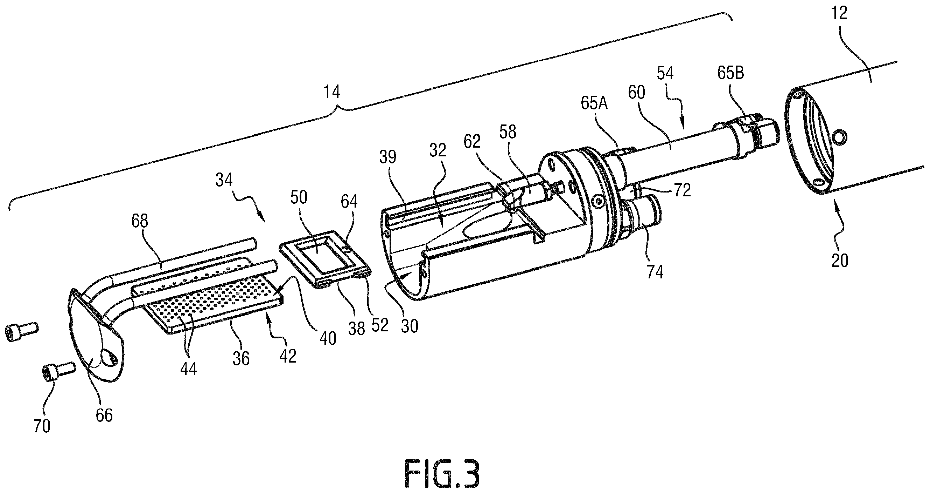

La tête 14 est une coque métallique creuse, représentée sur les figures 2 et 3, définissant un espace interne 30 débouchant vers l'extérieur par une ouverture 32. La tête 14 est fixée à la première extrémité 20 du manche 12 et s'étend sensiblement selon l'axe longitudinal X-X. La tête 14 présente une forme de cylindre tronqué suivant un plan passant par deux génératrices.

Par la suite, on entend par intérieurement et extérieurement des positions respectivement plus proches et plus éloignées par rapport à l'espace interne 30 de la tête 14.

L'ouverture 32 débouche dans l'espace interne 30 et à l'extérieur de la tête 14. L'ouverture 32 est par exemple rectangulaire, et présente une normale orientée de manière sensiblement perpendiculaire à l'axe longitudinal X-X.

La tête 14 définit, aux bords de l'ouverture 32, des rainures 39 parallèles à l'axe X-X débouchant à l'extrémité libre de la tête 14.

La tête 14 comprend un dispositif de filtration 34, destiné à empêcher le passage d'objets solides présentant une taille supérieure à un seuil de filtration à travers l'ouverture 32.

Le dispositif de filtration 34 comprend une paroi filtrante 36 disposée à travers l'ouverture 32 et une lame 38 de nettoyage adaptée pour nettoyer la paroi filtrante 36. Il comprend en outre un vérin 54 actionnant la lame 38, ainsi qu'un dispositif de maintien 56 de la lame 38 et de la paroi filtrante 36.

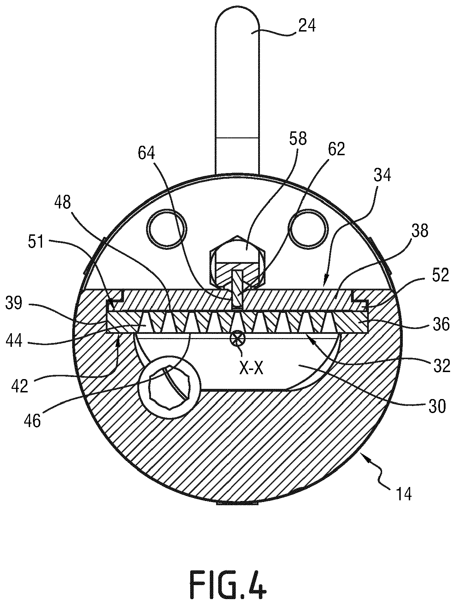

Comme représenté sur les figures 3 et 4, la paroi filtrante 36 et la lame 38 sont engagées dans les rainures 39 de la tête 14.

La paroi filtrante 36 est une plaque sensiblement rectangulaire présentant une face externe 40 et une face interne 42 ainsi qu'une pluralité d'orifices 44 traversant la paroi filtrante 36, débouchant sur la face externe 40 et sur la face interne 42.

Les orifices 44 présentent une première section transversale 46 située au voisinage de l'espace interne 30, par exemple au niveau de la face interne 42, et une deuxième section transversale 48 située à l'écart extérieurement de la première section transversale 46, par exemple au niveau de la face externe 40.

Dans l'exemple représenté, les orifices 44 présentent une forme tronconique, comportant une section étroite au niveau de la face externe 40 et une section plus large au niveau de la face interne 42. L'aire de la section large est supérieure à l'aire de la section étroite.

La géométrie des orifices 44 prévient l'accumulation de particules solides dans chaque orifice 44 au fur et à mesure de la circulation de fluide, et ainsi empêche que l'orifice 44 se bouche.

En référence à la figure 3, la lame 38 de nettoyage est adaptée pour parcourir la face externe 40 de la paroi filtrante 36 afin de retirer les particules solides qui s'y déposent. La lame 38 est de forme sensiblement rectangulaire, présentant une ouverture centrale 50.

L'ouverture centrale 50 augmente la longueur efficace de la lame 38 pour le nettoyage de la paroi filtrante 36. En effet, le nettoyage de la paroi filtrante 38 est effectué à la fois par un bord externe et par un bord interne de la lame 38 dans les deux sens de déplacement de la lame 38.

La lame 38 présente des surfaces de contact 51 sur une face inférieure, au niveau des bords latéraux de la lame 38. Par latéraux, on entend les bords de la lame 38 qui sont parallèles à la direction de déplacement.

Les surfaces de contact 51 sont des reliefs dépassant de la face inférieure de la lame 38, qui sont en appui sur la face supérieure 40 de la paroi filtrante 36. Ainsi la lame 38 n'est pas en contact direct avec la paroi filtrante 36 au niveau des bords transverses à la direction de déplacement.

La lame 38 présente également des protubérances 52 faisant saillie les bords latéraux de la lame 38.

Les protubérances 52 sont engagées dans les rainures 39, extérieurement par rapport à la paroi filtrante 36, de façon à guider le mouvement de la lame 38 en translation selon l'axe X-X, sur la paroi filtrante 36.

Le vérin 54 est un dispositif d'actionnement pneumatique apte à déplacer la lame 38 en translation le long de la face externe 40 de la paroi filtrante 36. Le vérin 54 comprend un bras 58 s'étendant parallèlement à l'axe X-X, et un piston 60 dans lequel le bras 58 est monté coulissant.

Le bras 58 est muni d'un ergot 62 à son extrémité libre, l'ergot 62 étant engagé dans une cavité 64 située sur la lame 38.

Le piston 60 est adapté pour mettre en mouvement le bras 58 selon l'axe X-X entre une position de retrait et une position d'extension. A cet effet, le piston 60 présente une prise avant 65A et une prise arrière 65B débouchant dans un espace intérieur du piston 60.

Les prises avant 65A et arrière 65B sont reliées fluidiquement à des conduites pneumatiques contenant de l'air comprimé, s'étendant à l'intérieur du manche 12 et débouchent dans l'espace intérieur du piston 60.

Les positions de retrait et d'extension correspondent aux fins de course du bras 58 dans le piston 60. En position d'extension, la lame 38 est plus éloignée du piston 60 qu'en position de retrait. La lame 38 s'étend en regard d'une première région de la paroi filtrante 36 et laisse découverte une deuxième région de la paroi filtrante 36. En position de retrait, la lame 38 s'étend en regard de la deuxième région et laisse au moins en partie découverte la première région.

La longueur du bras 58 est adaptée pour que la lame 38 n'entre pas en contact avec le dispositif de maintien 56 lorsque le bras 58 est en position d'extension.

L'application dans la prise avant 65A d'une pression supérieure à la pression dans la prise arrière 65B déplace le bras 58 vers sa position de retrait. A l'inverse, l'application dans la prise arrière 65B d'une pression supérieure à la pression dans la prise avant 65A déplace le bras 58 vers sa position d'extension.

Le dispositif de maintien 56 comprend un capot 66 et deux tiges de protection 68 rattachées au capot 66 par une extrémité respective.

Le capot 66 ferme l'espace interne 30 de la tête et présente deux orifices dans lesquels sont insérées deux vis de fixation 70 assemblant le dispositif de maintien à la tête de manière amovible.

Le dispositif de maintien 66 bloque la paroi filtrante 36 et la lame 38 dans les rainures 39 lorsqu'il est assemblé à la tête 14, et peut être désassemblé afin de démonter la paroi filtrante 36 et la lame 38 en vue de les nettoyer ou de les remplacer.

Les tiges de protection 68 s'étendent depuis le capot 66 extérieurement par rapport à la paroi filtrante 36 et à la lame 38, afin de les protéger des chocs extérieurs. Dans l'exemple représenté, les tiges de protection 68 s'étendent sensiblement parallèlement à l'axe X-X.

La conduite d'extraction 16 est avantageusement flexible. Elle est destinée à transporter le fluide depuis la tête 14 et le long du manche 12. La conduite d'extraction 16 s'étend à l'intérieur du manche 12. La conduite d'extraction 16 débouche dans l'espace interne 30 de la tête 14 à travers une prise d'extraction 72 par une première extrémité. Elle est apte à être reliée fluidiquement à une pompe d'extraction à une deuxième extrémité.

L'application d'une dépression dans la conduite d'extraction aspire le contenu de l'espace interne 30 et ainsi prélève du fluide depuis l'extérieur de la tête 14, à travers les orifices de la paroi filtrante 36.

La conduite de réinjection 18 est avantageusement flexible. Elle est destinée à transporter le fluide à travers le manche 12. La conduite de réinjection 18 s'étend à l'intérieur du manche 12. La conduite de réinjection est reliée fluidiquement à une prise de réinjection 74 par une première extrémité et est apte à être reliée fluidiquement à une pompe de réinjection à une deuxième extrémité.

La prise de réinjection 74 est située à l'entrée d'un canal de réinjection 76 passant à travers la tête 14.

Le canal de réinjection 76 est une perforation dans la tête 14, qui débouche à l'extérieur de la canne 10 par un orifice de sortie 78 situé dans la tête 14, du côté opposé à l'ouverture 32.

En fonctionnement, la canne 10 est intégrée à un système de prélèvement de fluide, qui comporte également une pompe d'extraction, une chambre de séparation des gaz, un dispositif d'analyse, et une pompe de réinjection.

La tête 14 de la canne 10 est plongée dans un fluide 100 à prélever. Le fluide 100 circule par exemple dans une conduite 102 selon un sens de circulation, comme représenté sur la figure 7.

La pompe d'extraction est reliée fluidiquement à la conduite d'extraction 16, et applique une dépression dans l'espace interne 30 de la tête 14 pour aspirer un échantillon de fluide à travers les orifices 44 de la paroi filtrante 36.

La chambre de séparation des gaz est propre à agiter l'échantillon de fluide prélevé pour en extraire les gaz.

Le dispositif d'analyse est relié fluidiquement à une sortie de séparation. Le dispositif d'analyse est adapté pour exécuter une ou plusieurs mesures sur les gaz dissous dans l'échantillon de fluide, par exemple mesurer le contenu du fluide en différents gaz.

La pompe de réinjection est reliée fluidiquement à une sortie du dispositif d'analyse et réinjecte l'échantillon de fluide analysé, à travers la conduite de réinjection 18 et l'orifice de sortie 78, dans la conduite 102.

La canne 10 est orientée dans la conduite 102 de façon à ce que la face externe 40 de la paroi filtrante 36 soit face au sens de circulation du fluide 100 dans la conduite 102, et à ce que l'orifice de sortie 78 soit situé en aval de la paroi filtrante 36 par rapport au sens de circulation. Ainsi, il n'y a pas de mesure en boucle d'un même échantillon de fluide, ce qui pourrait fausser les résultats des analyses.

Le fluide 100 est par exemple un fluide de forage, et la conduite 102 fait par exemple partie d'une installation de forage pétrolier. Le fluide 100 est analysé en sortie de puits, et contient des hydrocarbures dissous, dont on veut mesurer la concentration dans le fluide de forage 100.

La conduite 102 est alors avantageusement une conduite fermée, permettant d'empêcher l'émission de vapeurs inflammables sur le site de forage.

La canne 10, grâce à la forme tubulaire simple du manche 12, peut être insérée dans la conduite fermée 102 de manière étanche, par exemple grâce à un clapet 104 en deux parties, présentant un orifice central 106.

La canne 10 présente ainsi une forme compacte comprenant un unique tube 12, ce qui facilite son maniement et son insertion dans des espaces fermés. Le mouvement en translation de la lame 38 génère moins d'usure mécanique qu'un système rotatif classique. De plus, le dispositif de filtration 34 est entièrement et simplement démontable, ce qui facilite grandement l'entretien et le remplacement des différentes pièces.

Le vérin 54 fonctionne de manière uniquement pneumatique, la canne 10 ne nécessite pas de générer ou de transporter d'énergie électrique au voisinage de vapeurs d'hydrocarbures inflammables ou explosives, ce qui présente des avantages importants en termes de sécurité de l'installation de forage.

## Revendications

1. Canne (10) de prélèvement d'un fluide de forage comprenant :
- un manche (12) s'étendant selon un axe (X-X) ;
- une tête (14) de prélèvement située à une première extrémité (20) du manche (12), la tête (14) définissant un espace interne (30) débouchant vers l'extérieur par une ouverture (32), la tête (14) comprenant un dispositif de filtration (34) comprenant une paroi filtrante (36) disposée à travers l'ouverture (32) ; et
- une conduite d'extraction (16) s'étendant dans le manche (12) et débouchant dans l'espace interne (30) de la tête (14) ;
**caractérisée en ce que** le dispositif de filtration (34) comprend une lame (38) de nettoyage apte à parcourir une face externe (40) de la paroi filtrante (36), la lame (38) étant montée mobile en translation selon une direction parallèle à l'axe (X-X).

2. Canne (10) selon la revendication 1 dans laquelle le dispositif de filtration (34) comprend un vérin (54) apte à déplacer la lame (38) sur la face externe (40) de la paroi filtrante (36).

3. Canne (10) selon la revendication 2, dans laquelle le dispositif de filtration (34) comprend au moins une conduite pneumatique contenue dans le manche (12) et débouchant dans le vérin (54).

4. Canne (10) selon l'une quelconque des revendications précédentes, dans laquelle la lame (38) présente une ouverture centrale (50).

5. Canne (10) selon l'une quelconque des revendications précédentes, dans laquelle la lame (38) présente des surfaces de contact (51) avec la paroi filtrante (36) uniquement au niveau des bords latéraux de la lame (38).

6. Canne (10) selon l'une quelconque des revendications précédentes, dans laquelle la paroi filtrante (36) présente une pluralité d'orifices (44) présentant une première section transversale située au voisinage de l'espace interne (46), d'aire supérieure à l'aire d'une deuxième section transversale (48) située à l'écart extérieurement de la première section transversale (46).

7. Canne (10) selon la revendication 6, dans laquelle les orifices (44) présentent une forme tronconique.

8. Canne (10) selon l'une quelconque des revendications précédentes, dans laquelle la lame (38) et la surface filtrante (36) sont assemblées dans des rainures (39) de la tête (14), le dispositif de filtration (34) comprenant un dispositif de maintien (56) de la lame (38) et de la paroi filtrante (36), fixé de manière amovible à la tête (14).

9. Canne (10) selon l'une quelconque des revendications précédentes, dans laquelle la face externe (40) de la paroi filtrante (36) présente une normale sensiblement perpendiculaire à l'axe (X-X).

10. Canne (10) selon l'une quelconque des revendications précédentes, comprenant une conduite de réinjection (18) de fluide contenue dans le manche (12) et débouchant dans l'extérieur à travers un orifice de sortie (78) situé au niveau de la tête (14), à l'opposé de la paroi filtrante (36).

11. Système de prélèvement de fluide comportant :
- une canne (10) selon l'une quelconque des revendications précédentes, la tête (14) de la canne (10) étant destinée à être placée dans un fluide (100) à prélever ;
- une pompe d'extraction reliée fluidiquement à une extrémité libre de la conduite d'extraction (16) de la canne ;
- un dispositif d'analyse du fluide, relié fluidiquement à une sortie de la pompe d'extraction.

12. Système selon la revendication 11, dans lequel la canne (10) est selon la revendication 10, le système comprenant une pompe de réinjection reliée fluidiquement à la conduite de réinjection (18), l'orifice de sortie (78) étant situé en aval de la paroi filtrante (36) par rapport à un sens de circulation du fluide.

13. Installation de forage pétrolier comprenant un système selon l'une quelconque des revendications 11 et 12 et une conduite (102) dans laquelle circule un fluide (100) de forage, la tête (14) étant insérée dans la conduite (102) et immergée dans le fluide (100).

14. Installation selon la revendication 13, dans lequel la tête (14) est orientée dans la conduite (102) de façon à ce que la face externe (40) de la paroi filtrante (36) soit face à un sens de circulation du fluide (100) de forage.

15. Installation selon l'une quelconque des revendications 13 ou 14, comprenant une pompe d'extraction de fluide, raccordée à la conduite d'extraction (16), une chambre de séparation des gaz et un dispositif d'analyse des gaz récupérés dans la chambre de séparation.

## Patentansprüche

1. Stab (10) zur Entnahme eines Bohrfluids, welcher aufweist:
- einen Schaft (12), welcher sich entlang einer Achse (X-X) erstreckt,
- einen Kopf (14) zur Entnahme, welcher an einem ersten Ende (20) des Schafts (12) angeordnet ist, wobei der Kopf (14) einen Innenraum (30) definiert, welcher durch eine Öffnung (32) nach außen mündet, und wobei der Kopf (14) eine Filtervorrichtung (34) aufweist, welche eine Filterwand (36) aufweist, welche quer zur Öffnung (32) angeordnet ist, und
- eine Extraktionsleitung (16), welche sich im Schaft (12) erstreckt und welche in den Innenraum (30) des Kopfes (14) mündet,
**dadurch gekennzeichnet, dass** die Filtervorrichtung (34) eine Klinge (38) zur Reinigung aufweist, welche imstande ist, sich entlang einer Außenfläche (40) der Filterwand (36) zu bewegen, wobei die Klinge (38) entlang einer Richtung translationsbewegbar montiert ist, welche parallel zur Achse (X-X) ist.

2. Stab (10) gemäß Anspruch 1, wobei die Filtervorrichtung (34) einen Zylinder (54) aufweist, welcher imstande ist, die Klinge (38) an der Außenfläche (40) der Filterwand (36) zu bewegen.

3. Stab (10) gemäß Anspruch 2, wobei die Filtervorrichtung (34) mindestens eine Pneumatikleitung aufweist, welche im Schaft (12) enthalten ist und in den Zylinder (54) mündet.

4. Stab (10) gemäß irgendeinem der vorherigen Ansprüche, wobei die Klinge (38) eine zentrale Öffnung (50) hat.

5. Stab (10) gemäß irgendeinem der vorherigen Ansprüche, wobei die Klinge (38) Flächen für Kontakt (51) mit der Filterwand (36) ausschließlich auf dem Niveau von Lateralrändern der Klinge (38) hat.

6. Stab (10) gemäß irgendeinem der vorherigen Ansprüche, wobei die Filterwand (36) eine Mehrzahl von Öffnungen (44) hat, welche einen ersten Querschnitt haben, welcher sich benachbart zum Innenraum (46) befindet, mit einer Fläche, welche größer ist als die Fläche eines zweiten Querschnitts (48), welcher sich außen im Abstand zum ersten Querschnitt (46) befindet.

7. Stab (10) gemäß Anspruch 6, wobei die Öffnungen (44) eine kegelförmige Form haben.

8. Stab (10) gemäß irgendeinem der vorherigen Ansprüche, wobei die Klinge (38) und die Filterfläche (36) in Nuten (39) des Kopfes (14) zusammengefügt sind, wobei die Filtervorrichtung (34) eine Vorrichtung zum Halten (56) der Klinge (38) und der Filterwand (36) aufweist, welche auf abnehmbare Weise am Kopf (14) befestigt ist.

9. Stab (10) gemäß irgendeinem der vorherigen Ansprüche, wobei die Außenfläche (40) der Filterwand (36) eine Normale hat, welche im Wesentlichen senkrecht zur Achse (X-X) ist.

10. Stab (10) gemäß irgendeinem der vorherigen Ansprüche, welcher eine Leitung zum Wiedereinspritzen (18) von Fluid aufweist, welche im Schaft (12) enthalten ist und durch eine Ausgangsöffnung (78) nach außen mündet, welche sich auf dem Niveau des Kopfes (14) entgegengesetzt zur Filterwand (36) befindet.

11. System zur Entnahme von Fluid, welches aufweist:
- einen Stab (10) gemäß irgendeinem der vorherigen Ansprüche, wobei der Kopf (14) des Stabs (10) dazu bestimmt ist, in einem zu entnehmenden Fluid (100) angeordnet zu sein,
- eine Extraktionspumpe, welche mit einem freien Ende der Extraktionsleitung (16) des Stabs fluidverbunden ist,
- eine Vorrichtung zur Analyse des Fluids, welche mit einem Ausgang der Extraktionspumpe fluidverbunden ist.

12. System gemäß Anspruch 11, wobei der Stab (10) gemäß Anspruch 10 ist, und wobei das System eine Wiedereinspritzpumpe aufweist, welche mit der Wiedereinspritzleitung (18) fluidverbunden ist, wobei die Ausgangsöffnung (78) stromabwärts von der Filterwand (36) mit Bezug auf eine Strömungsrichtung des Fluids angeordnet ist.

13. Anordnung zum Ölbohren, welche ein System gemäß irgendeinem der Ansprüche 11 und 12 und eine Leitung (102) aufweist, in welcher ein Bohrfluid (100) strömt, wobei der Kopf (14) in die Leitung (102) eingeführt und in das Fluid (100) eingetaucht ist.

14. Anordnung gemäß Anspruch 13, wobei der Kopf (14) in der Leitung (102) auf eine Weise ausgerichtet ist, sodass die Außenfläche (40) der Filterwand (36) zu einer Strömungsrichtung des Bohrfluids (100) hin gewandt ist.

15. Anordnung gemäß irgendeinem der Ansprüche 13 oder 14, welche aufweist eine Pumpe zur Fluidextraktion, welche mit der Extraktionsleitung (16) verbunden ist, eine Kammer zum Trennen von Gasen und eine Vorrichtung zur Analyse von in der Kammer zum Trennen rekuperierten Gasen.

## Claims

1. Drilling fluid sampling probe (10) comprising:
- a handle (12) extending along an axis (X-X);
- a sampling head (14) located at a first end (20) of the handle (12), the head (14) defining an inner space (30) opening outwards through an opening (32), the head (14) comprising a filtration device (34) comprising a filtering wall (36) disposed through the opening (32); and
- an extraction duct (16) extending into the handle (12) and opening into the inner space (30) of the head (14);
**characterized in that** the filtration device (34) comprises a cleaning blade (38) designed to sweep an outer face (40) of the filtering wall (36), the blade (38) being mounted to move in translation in a parallel direction to the axis (X-X).

2. Probe (10) according to claim 1 wherein the filtration device (34) comprises a jack (54) designed to move the blade (38) over the outer face (40) of the filtering wall (36).

3. Probe (10) according to claim 2, wherein the filtration device (34) comprises at least one pneumatic duct contained in the handle (12) and opening into the jack (54).

4. Probe (10) according to any one of the preceding claims, wherein the blade (38) has a central opening (50).

5. Probe (10) according to any one of the preceding claims, wherein the blade (38) has contact surfaces (51) with the filtering wall (36) only at the lateral edges of the blade (38).

6. Probe (10) according to any one of the preceding claims, wherein the filtering wall (36) has a plurality of orifices (44) having a first cross-section adjacent the inner space (46), with an area greater than an area of a second cross-section (48) spaced apart from the first cross-section (46).

7. Probe (10) according to claim 6, wherein the orifices (44) have a frustoconical shape.

8. Probe (10) according to any one of the preceding claims, wherein the blade (38) and the filtering surface (36) are assembled in grooves (39) of the head (14), the filtering device (34) comprising a holding device (56) for the blade (38) and the filtering wall (36) removably attached to the head (14).

9. Probe (10) according to any one of the preceding claims, wherein the outer face (40) of the filtering wall (36) has a normal substantially perpendicular to the axis (X-X).

10. Probe (10) according to any one of the preceding claims comprising a fluid reinjection duct (18) contained in the handle (12) and opening into the outside through an outlet port (78) located at the head (14), opposite the filtering wall (36).

11. A fluid sampling system comprising:
- a probe (10) according to any one of the preceding claims, the head (14) of the probe (10) being intended to be placed in a fluid (100) to be sampled;
- an extraction pump, fluidly connected to a free end of the extraction duct (16) of the probe;
- a device for analyzing the fluid, fluidly connected to an outlet of the extraction pump.

12. System according to claim 11, wherein the probe (10) is according to claim 10, the system comprising a feedback pump fluidly connected to the reinjection duct (18), the outlet orifice (78) being located downstream of the filtering wall (36) with respect to a fluid flow direction.

13. Oil drilling installation comprising a system according to any one of claims 11 and 12 and a duct (102) in which a drilling fluid (100) circulates, the head (14) being inserted into the duct (102) and immersed in the fluid (100).

14. Installation according to claim 13, wherein the head (14) is so oriented in the duct (102) that the outer face (40) of the filtering wall (36) faces a flow direction of the drilling fluid (100).

15. Installation according to any one of claims 13 or 14, comprising a fluid extraction pump, connected to the extraction duct (16), a gas separation chamber, and a gas analysis device in the separation chamber.
